Europäisches Patentamt
European Patent Office
Office européen des brevets

(19)

(11) Veröffentlichungsnummer: **0 055 217 A2**

(12)

# EUROPÄISCHE PATENTANMELDUNG

(21) Anmeldenummer: **81810502.5**

(22) Anmeldetag: **16.12.81**

(51) Int. Cl.³: **C 07 D 501/00**, C 07 D 501/59

(30) Priorität: **22.12.80 CH 9468/80**

(43) Veröffentlichungstag der Anmeldung: **30.06.82 Patentblatt 82/26**

(84) Benannte Vertragsstaaten: **BE CH DE FR GB IT LI NL SE**


(71) Anmelder: **CIBA-GEIGY AG, Patentabteilung Postfach, CH-4002 Basel (CH)**

(72) Erfinder: **Sedelmeier, Gottfried, Dr., St. Gallusstrasse 10, D-7801 Ehrenkirchen-Norsingen (DE)**
Erfinder: **Scartazzini, Riccardo, Dr., Conrad Ferdinand Meyer-Strasse 38, CH-4059 Basel (CH)**


(54) **Verfahren zur Herstellung von 7-beta-Substituierten-3-unsubstituierten-3-cephem-4-carbonsäureverbindungen.**


(57) Es werden in einem neuen vorteilhaften Verfahren Verbindungen der Formel

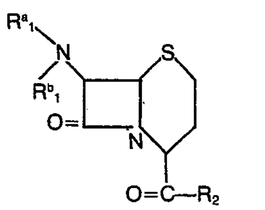

(I),

worin $R^a_1$ Wasserstoff oder eine Aminoschutzgruppe $R^A_1$. $R^b_1$ Wasserstoff oder eine Acylgruppe Ac oder $R^a_1$ und $R^b_1$ zusammen eine bivalente Aminoschutzgruppe und $R_2$ Hydroxy oder einen zusammen mit der Carbonylgruppierung –C(=O)– eine geschützte Carboxylgruppe bildenden Rest darstellen, sowie Salze von Verbindungen der Formel I mit salzbildenden Gruppen, durch Umsetzung der entsprechenden 3-Aminoverbindungen mit Borhydriden in Gegenwart von Säuren hergestellt.


EP 0 055 217 A2

0055217

CIBA-GEIGY AG
Basel (Schweiz)

4-13215/+

Verfahren zur Herstellung von 7β-Substituierten-3-unsubstituierten-3-cephem-4-carbonsäureverbindungen

Die vorliegende Erfindung betrifft ein neues, vorteilhaftes Verfahren zur Herstellung von 7β-Substituierten-3-unsubstituierten-3-cephem-4-carbonsäureverbindungen, die wertvolle antibiotisch wirksame Verbindungen oder wertvolle Zwischenprodukte zur Herstellung von antibiotisch wirksamen 7β-Substituierten-3-cephem-3-unsubstituierten-4-carbonsäureverbindungen sind. Die Erfindung betrifft ebenfalls die neuen 1-Oxide von 7β-Substituierten-3-amino-3-cephem-4-carbonsäureverbindungen, sowie Verfahren zur Herstellung dieser Verbindungen. Die Erfindung betrifft insbesondere ein Verfahren zur Herstellung von 7β-Substituierten-3-unsubstituierten-3-cephem-4-carbonsäureverbindungen durch Umsetzung von 7β-Substituierten-3-amino-3-cephem-4-carbonsäureverbindungen mittels geeigneter Borhydride in Gegenwart eines sauren Reagenzes.

Die Herstellung von 7β-Substituierten-3-cephem-3-unsubstituierten-4-carbonsäureverbindungen aus 7β-Substituierten-3-amino-4-carbonsäureverbindungen durch Umsetzung mit Diboran und anschliessendem Ansäuern ist in der Deutschen Offenlegungsschrift Nr. 26 20 308 beschrieben.

Gegenstand der vorliegenden Erfindung ist ein Verfahren zur Herstellung von 7β-Amino-3-cephem-4-carbonsäureverbindungen der Formel

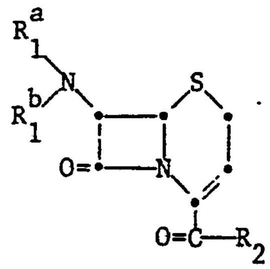

(I),

worin $R_1^a$ Wasserstoff oder eine Aminoschutzgruppe $R_1^A$, $R_1^b$ Wasserstoff oder eine Acylgruppe Ac oder $R_1^a$ und $R_1^b$ zusammen eine bivalente Aminoschutzgruppe und $R_2$ Hydroxy oder einen zusammen mit der Carbonylgruppierung -C(=O)- eine geschütze Carboxylgruppe bildenden Rest darstellen, sowie Salzen von Verbindungen der Formel I mit salzbildenden Gruppen, dadurch gekennzeichnet, dass man eine Verbindung der Formel

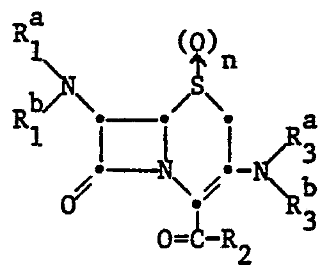

(II),

worin der Index n den Wert 0 oder 1 hat, $R_1^a$, $R_1^b$ und $R_2$ die unter Formel I genannten Bedeutungen haben und die Gruppe der Formel $-N(R_3^a)(R_3^b)$ für eine sekundäre oder tertiäre Aminogruppe steht, oder ein Salz davon mit einer geeigneten Borwasserstoffverbindung in Gegenwart eines sauren Reagenzes umsetzt und in an sich bekannter Weise, wenn erwünscht, eine erfindungsgemäss erhältliche Verbindung der Formel I in eine andere Verbindung der Formel I umwandelt und/oder in einer Verbindung der Formel I in geschützter Form vorliegende funktionelle Gruppen in die freien funktionellen Gruppen überführt und/oder ein erhältliches Salz in die freie Verbindung oder in ein anderes Salz überführt und/oder eine erhältliche freie Verbindung mit einer salzbildenden Gruppe in ein Salz überführt und/oder ein erhältliches Gemisch von isomeren Verbindungen der Formel I in die einzelnen Isomeren auftrennt.

In Verbindungen der Formel II, worin n den Wert 1 hat, liegt die

entsprechende 1-Oxidverbindung in der α- oder β-Form vor.

In der vorliegenden Beschreibung bedeutet der im Zusammenhang mit Definitionen von Substituenten oder Verbindungen verwendete Ausdruck "Nieder", zum Beispiel in Gruppen wie Niederalkyl, Niederalkylen, Niederalkoxy, Niederalkanoyl und dergleichen, dass die entsprechenden Gruppen, sofern nicht ausdrücklich anders definiert, bis zu 7, bevorzugt bis zu 4 C-Atome, enthalten.

Die in Verbindungen der Formel I oder II vorhandenen funktionellen Gruppen, insbesondere Carboxyl-, Amino-, Hydroxy-, Hydroxyimino- und Sulfogruppen, sind gegebenenfalls durch solche Schutzgruppen geschützt, die in der Penicillin-, Cephalosporin- und Peptidchemie verwendet werden.

Solche Schutzgruppen sind leicht, das heisst ohne dass unerwünschte Nebenreaktionen stattfinden, beispielsweise solovolytisch, reduktiv, photolytisch oder auch unter physiologischen Bedingungen abspaltbar.

Schutzgruppen dieser Art, sowie ihre Abspaltung sind beispielsweise beschrieben in "Protective Groups in Organic Chemistry", Plenum Press, London, New York, 1973, ferner in "The Peptides", Vol. I, Schröder and Lübke, Academic Press, London, New York, 1965, sowie in Houben-Weyl, Methoden der organischen Chemie, 4. Aufl., Bd. 15/1, Georg Thieme Verlag, Stuttgart, 1974.

Im Ausgangsmaterial der Formel II und im Produkt der Formel I haben die Gruppen $R_1^A$, $R_1^b$, Ac und $R_2$ beispielsweise die folgenden Bedeutungen: Eine Aminoschutzgruppe $R_1^A$ ist eine durch Wasserstoff ersetzbare Gruppe, in erster Linie eine Acylgruppe Ac, ferner eine Triarylmethylgruppe, sowie eine organische Silyl- oder Stannylgruppe.

Eine Acylgruppe Ac, die auch die Gruppe $R_1^b$ bedeuten kann, ist in erster Linie die Acylgruppe einer organischen Carbonsäure mit bis zu 18 und in erster Linie mit bis zu 10 Kohlenstoffatomen und ist insbesondere die Acylgruppe einer unsubstituierten oder substituierten aliphatischen, cycloaliphatischen, cycloaliphatisch-aliphatischen, aromatischen, araliphatischen, heterocyclischen oder heterocyclisch-aliphatischen Carbonsäure. Die Acylgruppe Ac steht insbesondere für eine Acylgruppe einer organischen Carbonsäure, welche in einem natürlich vorkommenden oder in einem bio-, halb- oder total-synthetisch herstellbaren, vorzugsweise pharmakologisch wirksamen, N-Acylderivat einer 6-Amino-penam-3-carbonsäure- oder 7-Amino-3-cephem-4-carbonsäureverbindung enthalten ist.

Eine solche Acylgruppe Ac ist in erster Linie eine Gruppe der Formel

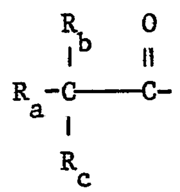

(IA),

worin (1) $R_a$ einen unsubstituierten oder substituierten carbocyclischen Arylrest, z.B. Phenyl, einen unsubstituierten oder substituierten, vorzugsweise ungesättigten cycloaliphatischen Kohlenwasserstoffrest, z.B. Cyclohexadienyl oder Cyclohexenyl, oder einen unsubstituierten oder substituierten heterocyclischen Arylrest, z.B. Thienyl, Furyl, Thiazolyl oder Thiadiazolyl, $R_b$ Wasserstoff und $R_c$ Wasserstoff oder unsubstituiertes oder substituiertes, insbesondere geschütztes, Hydroxy, Amino, Carboxyl oder Sulfo darstellen, oder worin (2) $R_a$ freies oder geschütztes $\omega$-Amino-$\omega$-carboxyniederalkyl, z.B. -propyl, Cyan, veräthertes Hydroxy oder Mercapto, z.B. unsubstituiertes oder substituiertes Phenyloxy, Phenylthio oder Pyridylthio, oder einen unsubstituierten oder substituierten, über ein Ringstickstoffatom verknüpften, ungesättigten heterocyclischen Rest, z.B. Tetrazolyl, und $R_b$ und $R_c$ Wasserstoff bedeuten.

Cyclohexadienyl ist insbesondere 1,4-Cyclohexadienyl, während Cyclohexenyl in erster Linie 1-Cyclohexenyl ist.

Thienyl ist vorzugsweise 2-, ferner 3-Thienyl. Furyl bedeutet insbesondere 2-Furyl Thiazolyl ist insbesondere 4-Thiazolyl. Thiadiazolyl ist insbesondere 1,2,4-Thiadiazolyl. Pyridylthio ist z.B. 4-Pyridylthio. Tetrazolyl ist z.B. 1-Tetrazolyl.

Substituenten eines Phenyl-, sowie eines Phenyloxyrests $R_a$, können in irgendeiner Stellung am Phenylring vorhanden sein und sind u.a. aliphatische Kohlenwasserstoffreste, z.B. unsubstituiertes oder substituiertes Niederalkyl, z.B. geschütztes Aminomethyl, freies oder funktionell abgewandeltes, z.B. veräthertes oder verestertes, Hydroxy oder unsubstituiertes oder substituiertes, insbesondere geschütztes, Amino, z.B. Acylamino, oder Nitro, das z.B. in der Phenyloxygruppe in 2-Stellung sein kann.

Substituenten eines Cyclohexadienyl- oder Cyclohexenylrests, sowie eines Thienyl- oder Furylrests $R_a$ sind z.B. unsubstituiertes oder substituiertes Niederalkyl, z.B. unsubstituiertes oder substituiertes, z.B. geschütztes Aminomethyl, wobei sich ein solcher Substituent, insbesondere freies oder geschütztes Aminomethyl, in erster Linie in 2-Stellung des 1,4-Cyclohexadienyl- oder 1-Cyclohexenylrestes oder in 5-Stellung des 2-Thienyl- oder 2-Furylrestes befindet. Substituiertes Thiazolyl ist insbesondere 2-Amino-4-thiazolyl und substituiertes Thiadiazolyl ist insbesondere 5-Amino-1,2,4-thiadiazolyl, worin die Aminogruppen frei oder geschützt oder durch Niederalkyl, insbesondere $C_1$-$C_4$-Niederalkyl, z.B. Methyl, Aethyl, Propyl, Isopropyl, Butyl oder tert.-Butyl, substituiert sind.

Freies oder geschütztes Aminomethyl ist in erster Linie unsubstituiertes oder durch Niederalkyl substituiertes Aminomethyl, z.B. Methylaminometyhl. Veräthertes Hydroxy ist beispielsweise Niederalkoxy, z.B.

Methoxy, und verestertes Hydroxy ist z.B. Niederalkanoyloxy, wie Acetyloxy, Aroyloxy, z.B. Benzoyloxy, Carbamoyloxy oder Halogen, z.B. Chlor. Unsubstituiertes oder substituiertes Amino ist z.B. durch Niederalkyl substituiertes Amino, z.B. Methylamino, oder Niederalkylsulfonylamino, z.B. Methylsulfonylamino.

Geschützte Hydroxy-, Amino-, Carboxyl- oder Sulfogruppen in Acylgruppen Ac der Formel IA sind solche Gruppen, die in der Penicillin- und Cephalosporinchemie üblich sind und die leicht in freie Hydroxy-, Amino-, Carboxyl- oder Sulfogruppen überführt werden können, ohne dass dabei das Cephemgerüst zerstört wird oder andere unerwünschte Nebenreaktionen stattfinden.

Aminogruppen können z.B. durch Acylgruppen geschützt sein, wobei eine solche Acylgruppe in erster Linie eine durch Reduktion, z.B. durch Behandeln mit einem chemischen Reduktionsmittel oder mit katalytisch aktiviertem Wasserstoff, oder durch Solvolyse, z.B. durch Behandeln mit einer geeigneten Säure, ferner auch mittels Bestrahlen, abspaltbare Acylgruppe eines Halbesters der Kohlensäure ist. Eine geschützte Aminogruppe ist vorzugsweise am ersten Kohlenstoffatom der veresternden Gruppe mehrfach verzweigtes und/oder durch Aryl, z.B. freies oder durch Niederalkoxy, z.B. Methoxy, und/oder Nitro substituiertes Phenyl oder Biphenyl, oder durch Arylcarbonyl, z.B. Benzoyl, substituiertes Niederalkoxycarbonyl, z.B. tert.-Butyloxycarbonyl, tert.-Pentyloxycarbonyl, Diphenylmethoxycarbonyl, 1-(4-Biphenyl)-1-methyläthoxycarbonyl, Benzyloxycarbonyl, 4-Nitrobenzyloxycarbonyl, 2-Nitro-4,5-dimethoxybenzyloxycarbonyl oder Phenacyloxycarbonyl, oder ist am zweiten Kohlenstoffatom der veresternden Gruppe durch Halogen substituiertes Niederalkoxycarbonyl, z.B. 2,2,2-Trichloräthoxycarbonyl oder 2-Jodäthoxycarbonyl, oder ist eine in letztere überführbare Gruppe, z.B. 2-Chlor- oder 2-Bromäthoxycarbonyl, oder ist ferner polycyclisches Cycloalkoxycarbonyl, z.B. Adamantyloxycarbonyl.

Eine Aminogruppe kann ferner durch Arylmethyl, wie Polyarylmethyl, z.B. Trityl, eine 2-Carbonylvinyl-Gruppierung, z.B. eine 1-Niederalkoxycarbonyl-1-propen-2-ylgruppe, z.B. 1-Methoxycarbonyl-1-propen-2-yl, durch eine Arylthio- oder Arylniederalkylthiogruppe, z.B. 2-Nitrophenylthio oder Pentachlorphenylthio, durch Tritylthio oder eine Arylsulfonylgruppe, ferner durch eine organische Silyl- oder Stannylgruppe, z.B. durch eine durch Niederalkyl, Halogenniederalkyl, Cycloalkyl, Phenyl oder Phenylniederalkyl, oder abgewandelte funktionelle Gruppen, wie Niederalkoxy, z.B. Methoxy, oder Halogen, z.B. Chlor, substituierte Silyl- oder Stannylgruppe, z.B. durch Triniederalkylsilyl, z.B. Trimethylsilyl, Halogenniederalkoxyniederalkylsilyl, z.B. Chlormethoxymethylsilyl, oder auch Triniederalkylstannyl, z.B. Tri-n-butylstannyl, geschützt sein.

Hydroxyschutzgruppen sind z.B. Acylgruppen, insbesondere die im Zusammenhang mit geschützten Aminogruppe genannten Acylgruppen von Kohlensäurehalbestern, oder sind organische Silyl- oder Stannylgruppen, ferner leicht abspaltbare 2-oxa- oder 2-thiaaliphatische oder -cycloalipahtische Kohlenwasserstoffreste. Hydroxyschutzgruppen sind in erster Linie 1-Niederalkoxyniederalkyl oder 1-Niederalkylthioniederalkyl, z.B. 1-Methoxyäthyl, 1-Aethoxyäthyl, 1-Methylthioäthyl oder 1-Aethylthioäthyl, oder 2-Oxa- oder 2-Thiacycloniederalkyl mit 5-7 Ringatomen, z.B. 2-Tetrahydrofuryl oder 2-Tetrahydropropanyl oder entsprechende Thiaanaloge, sowie leicht abspaltbare, unsubstituierte oder substituierte α-Phenylniederalkylreste, z.B. unsubstituiertes oder substituiertes Benzyl oder Diphenylmethyl, wobei als Substituenten der Phenylreste z.B. Halogen, wie Chlor, Niederalkoxy, wie Methoxy, und/oder Nitro in Frage kommen.

Eine geschützte Carboxyl- oder Sulfogruppe ist in erster Linie eine mit einem aliphatischen, cycloaliphatischen, cycloaliphatisch-aliphatischen, aromatischen oder araliphatischen Alkohol, wie einem Niederalkanol, oder mit einer Silyl- oder Stannylgruppe, wie Triniederalkylsilyl, veresterte Carboxyl- oder Sulfogruppe. In einer Carboxyl-oder Sulfogruppe kann die Hydroxygruppe beispielsweise wie die Hydroxygruppe in einer veresterten Carboxygruppe der Formel $-C(=O)-R_2$ veräthert sein.

Eine mit einer Aminoschutzgruppe $R_1^A$ geschützte Aminogruppe kann z.B. auch eine durch die Acylgruppe eines Kohlensäurehalbesters, eine 2-Carbonylvinyl-, Arylthio- oder Arylniederalkylthio- oder Arylsulfonylgruppe, eine Triarylmethylgruppe oder durch eine organische Silyl- oder Stannylgruppe geschützte Aminogruppe sein, wobei eine solche Schutzgruppe analog derjenigen einer entsprechend geschützten Aminogruppe in einer Acylgruppe der Formel IA sein kann.

Eine durch die Gruppen $R_1^a$ und $R_1^b$ zusammen gebildete bivalente Aminoschutzgruppe ist insbesondere die bivalente Acylgruppe einer organischen Dicarbonsäure, vorzugsweise mit bis zu 18 Kohlenstoffatomen, und ist in erster Linie die Diacylgruppe einer aliphatischen oder aromatischen Dicarbonsäure, beispielsweise die Diacylgruppe einer Niederalkan- oder Niederalkendicarbonsäure, wie Succinyl, oder einer -o-Arylendicarbonsäure, wie Phthaloyl, oder ist ferner die Acylgruppe einer, in α-Stellung vorzugsweise substituierten, z.B. einen aromatischen oder heterocyclischen Rest enthaltenden α-Aminoessigsäure, worin die Aminogruppe über einen, vorzugsweise substituierten, z.B. zwei Niederalkyl-, wie Methylgruppen, enthaltenden Methylenrest mit dem Stickstoffatom verbunden ist, z.B. ein, insbesondere in 2-Stellung, substituierter, z.B. gegebenenfalls substituiertes Phenyl oder Thienyl enthaltender, und in 4-Stellung gegebenenfalls durch Niederalkyl, wie Methyl-, mono- oder di-substituierter 1-Oxo-3-aza-1,4-butylenrest, z.B. 4,4-Dimethyl-2-phenyl-1-oxo-3-aza-1,4-butylen.

Die Gruppen $R_1^a$ und $R_1^b$ können zusammen auch eine organische, z.B. eine aliphatische, cycloalipahtische, cycloaliphatisch-aliphatische oder araliphatische Ylidengruppe, vorzugsweise mit bis zu 18 Kohlenstoffatomen, darstellen.

Eine geschützte Carboxylgruppe der Formel $-C(=O)-R_2$ ist in erster Linie eine veresterte Carboxylgruppe, worin $R_2$ eine durch einen organischen Rest oder eine organische Silyl- oder Stannylgruppe substituierte Hydroxygruppe darstellt. Solche organischen Reste, auch als Substituenten in organischen Silyl- oder Stannylgruppen, sind aliphatische, cycloaliphatische, cycloaliphatisch-aliphatische, aromatische oder araliphatische Reste, insbesondere unsubstituierte oder substituierte Kohlenwasserstoffreste dieser Art, sowie heterocyclische oder heterocyclisch-aliphatische Reste, vorzugsweise mit bis zu 18 Kohlenstoffatomen.

Eine substituierte Hydroxygruppe $R_2$ bildet zusammen mit der Carbonylgruppierung eine vorzugsweise leicht spaltbare, z.B. reduktiv, z.B. hydrogenolytisch oder solvolytisch, z.B. acidolytisch oder hydrolytisch, sowie oxidativ spaltbare, oder leicht in eine andere funktionell abgewandelte Carboxylgruppe, z.B. in eine andere veresterte Carboxylgruppe oder in eine Hydrazinocarbonylgruppe, umwandelbare, veresterte Carboxylgruppe. Eine solche Gruppe $R_2$ ist z.B. 2-Halogenniederalkoxy, worin Halogen vorzugsweise ein Atomgewicht höher als 19 hat, z.B. 2,2,2-Trichloräthoxy oder 2-Jodäthoxy, ferner 2-Chloräthoxy oder 2-Bromäthoxy, das sich leicht in letzteres überführen lässt, oder 2-Niederalkylsulfonylniederalkoxy, z.B. 2-Methylsulfonyläthoxy. Die Gruppe $R_2$ ist ferner eine durch unsubstituierte oder substituierte Kohlenwasserstoffreste, insbesondere gesättigte aliphatische oder aromatische Kohlenwasserstoffreste, wie Niederalkyl, z.B. Methyl und/oder Phenyl, polysubstituierte Methoxygruppe oder ist eine durch einen ungesättigten aliphatischen Kohlenwasserstoffrest, wie Niederalkenyl, z.B. 1-Niederalkenyl, wie Vinyl, oder durch eine Elektronen-abgebende, Substituenten aufweisende, carbocyclische Arylgruppe oder durch eine, Sauerstoff oder Schwefel als Ringglied aufweisende, heterocyclische Gruppe aromatischen Charakters, monosubstituierte Methoxygruppe, beispielsweise tert.-Niederalkoxy, z.B. tert.-Butyloxy oder tert.-Pentyloxy, unsubstituiertes oder substituiertes Diphenylmethoxy, z.B. Diphenylmethoxy oder 4,4'-Dimethoxydiphenylmethoxy, Niederalkenyloxy, insbesondere 2-Niederalkenyloxy, z.B. Allyloxy, Niederalkoxyphenylniederalkoxy, z.B. Niederalkoxybenzyloxy, wie Methoxybenzyloxy (wobei Methoxy in erster Linie in 2-, 4- und/oder 5-Stellung steht), in erster Linie 3- oder 4-Methoxybenzyloxy, 3,4-Dimethoxybenzyloxy, oder vor allem Nitrobenzyloxy, z.B. 4-Nitrobenzyloxy, 2-Nitrobenzyloxy oder 4,5-Dimethoxy-2-nitrobenzyloxy, sowie Furfuryloxy, wie 2-Furfuryloxy. $R_2$ kann auch 2-Oxa- oder 2-Thiacycloalkoxy oder -cycloalkenyloxy mit 5-7 Ringgliedern, wie 2-Tetrahydrofuryloxy, 2-Tetrahydropyranyloxy oder 2,3-Dihydro-2-pyranyloxy oder eine entsprechende Thiagruppe, oder Arylcarbonylmethoxy, worin Aryl insbesondere für eine unsubstituierte oder substituierte Phenylgruppe steht, z.B. Phenacyloxy, sein oder $R_2$ bildet zusammen mit der -C(=O)-Gruppierung eine aktivierte Estergruppe und ist beispielsweise Nitrophenyloxy, z.B. 4-Nitrophenyloxy oder 2,4-Dinitrophenyloxy, oder Polyhalogenphenyloxy, z.B. Pentachlorphenyloxy. $R_2$ kann aber auch unverzweigtes Niederalkoxy, z.B. Methoxy oder Aethoxy, sein.

Eine organische Silyloxy- oder Stannyloxygruppe $R_2$ ist insbesondere eine durch 1 bis 3 unsubstituierte oder substituierte Kohlenwasserstoffreste, vorzugsweise mit bis zu 18 Kohlenstoffatomen, substituierte Silyloxy- oder Stannyloxygruppe. Sie enthält als Substituenten vorzugsweise unsubstituierte oder substituierte, beispielsweise durch Niederalkoxy, wie Methoxy, oder durch Halogen, wie Chlor, substituierte aliphatische, cycloaliphatische, aromatische oder araliphatische Kohlenwasserstoffreste, wie Niederalkyl, Halogenniederalkyl, Cycloalkyl, Phenyl oder Phenylniederalkyl, und stellt in erster Linie Triniederalkylsilyloxy, z.B. Trimethylsilyloxy, Halogenniederalkoxyniederalkylsilyloxy, z.B. Chlormethoxymethylsilyloxy, oder Triniederalkylstannyloxy, z.B. Tri-n-butylstannyloxy, dar.

Die Gruppe $R_2$ kann auch eine substituierte Hydroxygruppe sein, die zusammen mit der Carbonylgruppierung -C(=O)- eine unter physiolo-

gischen Bedingungen spaltbare veresterte Carboxylgruppe bildet, und ist in erster Linie eine Acyloxymethoxygruppe, worin Acyl z.B. den Rest einer organischen Carbonsäure, in erster Linie den Rest einer gegebenenfalls substituierten Niederalkancarbonsäure, bedeutet, oder worin Acyloxymethyl den Rest eines Lactons bildet. Solche Hydroxygruppen sind Niederalkanoyloxymethoxy, z.B. Acetyloxymethyloxy oder Pivaloyloxymethoxy, Aminoniederalkanoyloxymethoxy, insbesondere α-Aminoniederalkanoyloxymethoxy, z.B. Glycyloxymethoxy, L-Valyloxymethoxy, L-Leucyloxymethoxy, ferner Phthalidyloxy.

Ein zusammen mit einer -C(=O)-Gruppierung eine gegebenenfalls substituierte Hydrazinocarbonylgruppe bildender Rest $R_2$ ist z.B. Hydrazino oder 2-Niederalkylhydrazino, z.B. 2-Methylhydrazino.

Salze sind insbesondere diejenigen von Verbindungen der Formel I oder II, die eine saure Gruppierung besitzen, z.B. eine Carboxygruppe, und sind in erster Linie Metall- oder Ammoniumsalze, beispielsweise Alkalimetall- und Erdalkalimetall-, z.B. Natrium-, Kalium-, Magnesium- oder Calciumsalze, sowie Ammoniumsalze, welche mit Ammoniak oder geeigneten organischen Aminen gebildet werden, wobei in erster Linie aliphatische, cycloaliphatische, cycloaliphatisch-aliphatische und araliphatische primäre, sekundäre oder tertiäre Mono-, Di- oder Polyamine, sowie heterocyclische Basen für die Salzbildung in Frage kommen, beispielsweise Niederalkylamine, z.B. Triäthylamin, Hydroxyniederalkylamine, z.B. 2-Hydroxyäthylamin, Di-(2-hydroxyäthyl)-amin oder Tri-(2-hydroxyäthyl)-amin, ferner basische aliphatische Ester von Carbonsäuren, z.B. 4-Aminobenzoesäure-2-diäthylaminoäthylester, Niederalkylenamine, z.B. 1-Aethylpiperidin, Cycloalkylamine, z.B. Bicyclohexylamin oder Benzylamine, z.B. N,N'-Dibenzyläthylendiamin, sowie Basen vom Pyridintyp, z.B. Pyridin, Collidin oder Chinolin. Verbindungen der Formel I oder II, die eine basische Gruppe aufweisen, können ebenfalls Säureadditionssalze, z.B. mit anorganischen Säuren, wie Salzsäure, Schwefelsäure oder Phosphorsäure, oder mit geeigneten

organischen Carbon- oder Sulfonsäuren, z.B. Trifluoressigsäure oder p-Toluolsulfonsäure, bilden. Verbindungen der Formel I oder II mit einer sauren und einer basischen Gruppe können auch in Form von inneren Salzen, d.h. in zwitterionischer Form, vorliegen. 1-Oxide von Verbindungen der Formel II mit salzbildenden Gruppen können ebenfalls Salze, wie oben beschrieben, bilden. In einem Ausgangsmaterial der Formel II sind diejenigen Salze bevorzugt, die die Reduktion nicht stören.

In einem Ausgangsmaterial der Formel II bedeutet in einer sekundären Aminogruppe $-N(R_3^a)(R_3^b)$ einer der Substituenten $R_3^a$ und $R_3^b$ Wasserstoff und der andere einen aliphatischen oder cycloalipahtischen Kohlenwasserstoffrest, der etwa bis zu 18, insbesondere bis zu 12 und bevorzugt bis zu 7, Kohlenstoffatome enthält. Aliphatische Kohlenwasserstoffreste $R_3^a$ oder $R_3^b$ sind beispielsweise unsubstituierte oder substituierte, z.B. durch Niederalkoxy, wie Methoxy, Niederalkylthio, wie Methylthio, Cycloalkyl, wie Cyclohexyl, Aryl, wie Phenyl oder Heterocyclyl, wie Thienyl, substituierte Alkyl-, insbesondere Niederalkylreste, z.B. Methyl, Aethyl, Propyl, Isopropyl, Butyl, Isobutyl, Pentyl, Hexyl, 2-Aethoxyäthyl, 2-Methylthioäthyl, Cyclohexylmethyl, Benzyl oder Thienylmethyl. Cycloaliphatische Kohlenwasserstoffreste $R_3^a$ oder $R_3^b$ sind beispielsweise unsubstituierte oder substituierte, z.B. durch Niederalkyl, wie Methyl, Niederalkoxy, wie Methoxy, Niederalkylthio, wie Methylthio, Cycloalkyl, wie Cyclohexyl, Aryl, wie Phenyl oder Heterocyclyl, wie Furyl, substituierte Cycloalkylreste, z.B. unsubstituiertes oder wie angegeben substituiertes Cyclopropyl, Cyclopentyl, Cyclohexyl oder Cycloheptyl.

In tertiären Aminogruppen $-N(R_3^a)(R_3^b)$ bedeutet jeder der Substituenten $R_3^a$ und $R_3^b$ einen der angegebenen aliphatischen oder cycloaliphatischen Kohlenwasserstoffreste, wobei $R_3^a$ und $R_3^b$ gleich oder verschieden sein können, und wobei beide Substituenten $R_3^a$ und $R_3^b$ durch eine Kohlenstoff-Kohlenstoff-Bindung oder über ein Sauerstoff-, Schwefel- oder gegebenenfalls substituiertes, wie niederalkyliertes, z.B. methyliertes, Stickstoffatom miteinander verbunden sein können.

Geeignete tertiäre Aminogruppen $N(R_3^a)(R_3^b)$ sind beispielsweise Dimethylamino, Diäthylamino, N-Methyläthylamino, Diisopropylamino, N-Methylisopropylamino, Dibutylamino, N-Methylisobutylamino, Dicyclopropylamino, N-Methylcyclopropylamino, Dicyclopentylamino, N-Methylcyclopentylamino, Dicyclohexylamino, N-Methylcyclohexylamino, Dibenzylamino, N-Methylbenzylamino, N-Cyclopropylamino, 1-Aziridinyl, 1-Pyrrolidinyl, 1-Piperidyl, 1H-2,3,4,5,6,7-Hexahydroazepinyl, 4-Morpholinyl, 4-Thiomorpholinyl, 1-Piperazinyl oder 4-Methyl-1-piperazinyl.

In einem Produkt der Formel I ist eine Acylgruppe Ac ebenfalls eine Gruppe der Formel Ia, worin (3) $R_a$ einen unsubstituierten oder substituierten carbocyclischen Arylrest, z.B. Phenyl, oder einen unsubstituierten oder substituierten heterocyclischen Arylrest, z.B. Thienyl, Furyl, Thiazolyl oder Thiadiazolyl und $R_b$ und $R_c$ zusammen vorzugsweise O-substituiertes Hydroxyimino in der syn-Konfiguration bedeuten.

O-substituiertes Hydroxyimino ist insbesondere Niederalkoxyimino, z.B. Methoxyimino oder Aethoxyimino, ferner Phenyloxyimino oder Phenylniederalkoxyimino, z.B. Benzyloxyimino, wobei solche Gruppen vorzugsweise in der syn-Form vorliegen.

Die Erfindung betrifft insbesondere ein Verfahren zur Herstellung von Verbindungen der Formel I und Salzen von solchen Verbindungen mit salzbildenden Gruppen aus entsprechend substituierten Verbindungen der Formel II, deren 1-Oxiden oder Salzen, worin $R_1^a$ Wasserstoff oder eine Acylgruppe Ac, welche in einem fermentativ (d.h. natürlich vorkommenden) oder bio-, halb- oder totalsynthetisch herstellbaren N-Acylderivat einer 6β-Amino-penam-3-carbonsäure- oder 7β-Amino-3-cephem-4-carbonsäureverbindung enthalten ist, z.B. eine oben genannte Acylgruppe der Formel IA, bedeutet, worin $R_a$, $R_b$ und $R_c$ die hervorgehobenen Bedeutungen haben, $R_1^b$ für Wasserstoff steht, oder worin

$R_I^a$ und $R_1^b$ zusammen einen in 2-Stellung durch einen aromatischen oder heterocyclischen Rest, z.B. Phenyl, und in 4-Stellung durch zwei Niederalkylgruppen, wie Methyl, substituierten 1-Oxo-3-aza-1,4-butylenrest darstellen und $R_2$ für Hydroxy, eine durch einen organischen Rest oder eine organische Silyl- oder Stannylgruppe substituierte Hydroxygruppe oder für eine unsubstituierte oder substituierte Hydrazinogruppe steht.

In erster Linie steht in einer Verbindung der Formel I oder II oder in einem Salz einer solchen Verbindung mit salzbildenden Gruppen $R_1^a$ für Wasserstoff oder eine Acylgruppe der Formel IA steht, worin (1) $R_a$ in erster Linie die hervorgehobenen Bedeutungen hat, und beispielsweise unsubstituiertes oder durch Hydroxy, geschütztes Hydroxy, Niederalkoxy, Niederalkanoyloxy, Carbamoyloxy, Halogen, Niederalkylsulfonylamino oder Aminomethyl substituiertes Phenyl, Thienyl, Furyl, Cyclohexadienyl oder Cyclohexenyl oder durch Amino, Niederalkylamino oder geschütztes Amino substituiertes Thiazolyl oder Thiadiazolyl, $R_b$ Wasserstoff und $R_c$ Wasserstoff, freies oder geschütztes Hydroxy, freies oder geschütztes Amino oder freies oder geschütztes Carboxyl oder Sulfo darstellen, oder worin (2) $R_a$ freies oder geschütztes 3-Amino-3-carboxypropyl, Cyan, 1-Tetrazolyl, unsubstituiertes oder wie Phenyl substituiertes Phenyloxy oder 4-Pyridylthio und $R_b$ und $R_c$ Wasserstoff darstellen, $R_1^b$ für Wasserstoff steht und $R_2$ Hydroxy, Niederalkoxy oder $\alpha$-polyverzweigtes Niederalkoxy, z.B. Methoxy oder tert.-Butyloxy, oder 2-Halogenniederalkoxy, z.B. 2,2,2-Trichloräthoxy, 2-Jodäthoxy oder das leicht in dieses überführbare 2-Chloräthoxy oder 2-Bromäthoxy, ferner Phenacyloxy, 1-Phenylniederalkoxy mit 1-3 unsubstituierten oder durch Niederalkoxy und/oder Nitro substituierten Phenylresten, z.B. 4-Methoxybenzyloxy, 4-Nitrobenzyloxy, 2-Nitro-4,5-dimethoxybenzyloxy, Diphenylmethoxy, 4,4'-Dimethoxydiphenylmethoxy oder Trityloxy, Niederalkanoyloxymethoxy, z.B. Acetyloxymethoxy oder Pivaloyloxymethoxy, $\alpha$-Aminoniederalkanoyloxymethoxy, z.B. Glycyloxymethoxy, 2-Phthalidyloxy, sowie Niederalkenyloxy, insbesondere 2-Niederalkenyloxy, z.B. Allyloxy, bedeutet.

Insbesondere steht ebenfalls in einer Verbindung der Formel II $R_1^a$ für eine Acylgruppe der Formel IA, worin (3) $R_a$ Phenyl, Thienyl, Furyl oder durch Amino, Niederalkylamino oder geschütztes Amino substituiertes Thiazolyl oder Thiadiazolyl darstellt und $R_b$ und $R_c$ zusammen syn-Niederalkoxyimino bedeuten.

Die Erfindung betrifft in erster Linie ein Verfahren zur Herstellung von Verbindungen der Formel I und Salzen von solchen Verbindungen mit salzbildenden Gruppen aus entsprechend substituierten Verbindungen der Formel II, deren 1-Oxiden oder Salzen, worin $R_1^a$ Wasserstoff oder eine Acylgruppe der Formel IA darstellt, worin (1) $R_a$ Phenyl, Hydroxyphenyl, z.B. 3- oder 4-Hydroxyphenyl, Niederalkylsulfonylaminophenyl, z.B. 3-Methylsulfonylaminophenyl, Aminomethylphenyl, z.B. 2-Aminomethylphenyl, Thienyl, z.B. 2- oder 3-Thienyl, Aminomethylthienyl, z.B. 5-Aminomethyl-2-thienyl, Furyl, z.B. 2-Furyl, Aminomethylfuryl, z.B. 5-Aminomethyl-2-furyl, Cyclohexadienyl, z.B. 1,4-Cyclohexadienyl, Aminomethyl-1,4-cyclohexadienyl, z.B. 2-Aminomethyl-1,4-cyclohexydienyl, Cyclohexenyl, z.B. 1-Cyclohexenyl, Aminomethyl-1-cyclohexenyl, z.B. 2-Aminomethyl-1-cyclohexenyl, Aminothiazolyl, z.B. 2-Amino-4-thiazolyl, Niederalkylaminothiazolyl, z.B. 2-Methylamino-4-thiazolyl, Aminothiadiazolyl, z.B. 5-Amino-1,2,4-thiadiazolyl, oder Niederalkylaminothiadiazolyl, z.B. 5-Methylamino-1,2,4-thiadiazolyl, wobei in den obigen Resten Hydroxy und/oder Amino unsubstituiert oder beispielsweise durch Acyl, z.B. gegebenenfalls halogeniertes Niederalkoxycarbonyl, z.B. tert.-Butyloxycarbonyl oder 2,2,2-Trichloräthoxycarbonyl, geschützt sind, $R_b$ für Wasserstoff und $R_c$ für Wasserstoff, Amino, geschütztes Amino, beispielsweise Acylamino, z.B. β-polyverzweigtes Niederalkoxycarbonylamino, z.B. tert.-Butyloxycarbonylamino oder 2-Halogenniederalkoxycarbonylamino, z.B. 2,2,2-Trichloräthoxycarbonylamino, 2-Jodäthoxycarbonylamino oder 2-Bromäthoxycarbonylamino, oder unsubstituiertes oder durch Niederalkoxy- und/oder Nitro substituiertes Phenylniederalkoxycarbonylamino, z.B. 4-Methoxybenzyloxycarbonylamino oder Diphenylmethoxycarbonylamino, Hydroxy,

geschütztes Hydroxy, beispielsweise Acyloxy, z.B. β-polyverzweigtes Niederalkoxycarbonyl, wie tert.-Butyloxycarbonyloxy, oder 2-Halogenniederalkoxycarbonyloxy, wie 2,2,2-Trichloräthoxycarbonyloxy, 2-Jodäthoxycarbonyloxy oder 2-Bromäthoxycarbonyloxy, oder für freies oder mit Niederalkyl, z.B. Methyl, verestertes Carboxyl oder Sulfo stehen, oder worin (2) $R_a$ 3-Amino-3-carboxypropyl, worin Amino z.B. wie die obige Aminogruppe $R_c$ und Carboxy z.B. wie die 4-Carboxygruppe $-C(=O)-R_2$ geschützt sein können, Cyan, 1-Tetrazolyl, Phenyloxy oder 4-Pyridylthio und $R_b$ und $R_c$ Wasserstoff darstellen, $R_1^b$ Wasserstoff bedeutet, und $R_2$ für Hydroxy, Methoxy, α-polyverzweigtes Niederalkoxy, z.B. tert.-Butyloxy, 2-Halogenniederalkoxy, z.B. 2,2,2-Trichloräthoxy, 2-Jodäthoxy oder 2-Bromäthoxy, unsubstituiertes oder durch Niederalkoxy, wie Methoxy, substituiertes Diphenylmethoxy, z.B. Diphenylmethoxy oder 4,4'-Dimethoxydiphenylmethoxy, 4-Nitrobenzyloxy, Triniederalkylsilyloxy, z.B. Trimethylsilyloxy, sowie 2-Niederalkenyloxy, z.B. Allyloxy, steht.

In erster Linie steht ebenfalls in einer Verbindung der Formel I $R_1^a$ für eine Acylgruppe der Formel IA, worin (3) $R_a$ Phenyl, 2-Thienyl, 2-Furyl, 2-Amino-4-thiazolyl, 2-Niederalkylamino-, z.B. 2-Methylamino-4-thiazolyl, 5-Amino-1,2,4-thiadiazolyl oder 5-Niederalkylamino-, z.B. 5-Methylamino-1,2,4-thiadiazolyl, worin Amino z.B. durch Acyl, z.B. gegebenenfalls halogeniertes Niederalkoxycarbonyl, z.B. tert.-Butyloxycarbonyl oder 2,2,2-Trichloräthoxycarbonyl, geschützt sein kann, darstellen und $R_b$ und $R_c$ zusammen syn-Niederalkoxyimino, z.B. syn-Methoxyimino, bedeuten.

Die Erfindung betrifft vor allem ein Verfahren zur Herstellung von Verbindungen der Formel I und Salzen von solchen Verbindungen mit salzbildenden Gruppen aus entsprechend substituierten Verbindungen der Formel II, deren 1-Oxiden oder Salzen, sowie 1-Oxide von Verbindungen der Formel II und Salze von solchen Verbindungen mit salzbildenden Gruppen, worin $R_1^a$ Wasserstoff oder eine Acylgruppe der Formel IA darstellt, worin (1) $R_a$ für Phenyl, 2- oder 3-Thienyl, 2-

oder 3-Furyl, $R_b$ für Wasserstoff, freies oder, z.B. wie oben beschrieben, geschütztes Amino und $R_c$ für Wasserstoff stehen, oder worin (2) $R_a$ für Phenyloxy und $R_b$ und $R_c$ je für Wasserstoff stehen, und $R_2$ Hydroxy, eine veresterte Hydroxygruppe, beispielsweise Niederalkoxy, z.B. Methoxy oder tert.-Butoxy, 2-Halogenniederalkoxy, z.B. 2,2,2-Trichloräthoxy, Nitrobenzyloxy, z.B. 4-Nitrobenzyloxy, oder Diphenylmethoxy bedeutet.

Vor allem steht ebenfalls in einer Verbindung der Formel I $R_1^a$ für eine Acylgruppe der Formel IA, worin (3) $R_a$ Phenyl, 2-Amino-4-thiazolyl, 2-Niederalkylamino-, z.B. 2-Methylamino-4-thiazolyl, 5-Amino-1,2,4-thiadiazolyl oder 5-Niederalkylamino, z.B. 5-Methylamino-1,2,4-thiadiazolyl, worin Amino z.B. durch Acyl, wie gegebenenfalls halogeniertes Niederalkoxycarbonyl, z.B. tert.-Butyloxycarbonyl oder 2,2,2-Trichloräthoxycarbonyl, geschützt sein kann, darstellt, und $R_b$ und $R_c$ zusammen syn-Niederalkoxyimino, z.B. syn-Methoxyimino, bedeuten.

Die für das erfindungsgemässe Verfahren geeigneten Borwasserstoffverbindungen sind beispielsweise komplexe Borhydride, z.B. salzartige komplexe Borhydride, z.B. Metallborhydride wie Zinkborhydrid, Alkalimetallborhydride, z.B. Natrium- oder Kaliumborhydrid, oder solche komplexe Borhydride, worin ein bis drei Wasserstoffatome durch Cyano-, Acyloxygruppen, beispielsweise durch gegebenenfalls substituierte Alkanoyloxygruppen, z.B. Formyloxy, Acetyloxy, Propionyloxy, Palmityloxy, Monochloracetyloxy oder Trifluoracetyloxy, oder durch aromatische Acyloxygruppen, z.B. Benzyloxy, durch Niederalkoxy, z.B. Aethoxy oder Isopropoxy, oder durch Alkylreste, z.B. n-Propyl, n-Butyl, Isopropyl oder sec-Butyl, ersetzt sind, z.B. Lithiumcyanborhydrid ($LiBH_3CN$), Natriumcyanborhydrid, Natriumtriacetyloxyborhydrid ($NaBH(CH_3COO)_3$), Natriumtriäthoxyborhydrid, Kaliumtriisopropoxyborhydrid, Lithium-tri-sec.-butylborhydrid, Kalium-tri-sec.-butylbor-

hydrid, sowie Ammoniumborhydride, worin das Ammoniumion gegebenenfalls durch 1-4 Alkylgruppen, z.B. n-Butylgruppen, und das Borhydridanion gegebenenfalls durch ein bis drei der oben genannten Reste substituiert ist, z.B. Tetrabutylammoniumborhydrid ($(C_4H_9)_4NBH_4$) oder Tetrabutylammoniumcyanborhydrid ($(C_4H_9)_4NBH_3CN$) oder Borwasserstoffkomplexe mit primären, sekundären oder tertiären Aminen oder mit Diorganylsulfiden, z.B. t.Butylaminboran, Dimethylaminboran, Trimethylaminboran, Triäthylaminboran oder Dimethylsulfidboran. Geeignete Borwasserstoffverbindungen sind ebenfalls Monoalkylborane mit einem verzweigten Alkylrest mit ca. 5-10 Kohlenstoffatomen, z.B."Thexylboran"($(CH_3)_2CH-C(CH_3)_2BH$), Dialkylborane, worin die beiden Alkylgruppen untereinander verbunden sind, z.B. "Borabicyclononan" (9-BBN) hergestellt aus Cycloocta-1,5-dien und Diboran. Das Borhydrid wird, bezogen auf das Ausgangsmaterial der Formel II, in mindestens äquivalenter Menge, bevorzugt in einer Menge von 1 bis 10 Aequivalenten, bei Verwendung des S-Oxids der Formel II bevorzugt in einer Menge von 2 bis 10 Aequivalenten, gegebenenfalls portionenweise, zugesetzt.

Bevorzugte komplexe Borhydride sind beispielsweise Alkalimetallborhydride, z.B. Natrium- oder Kaliumborhydrid, Natriumcyanborhydrid und Natriumtriacetyloxyborhydrid.

Letzteres lässt sich beispielsweise in situ aus Natriumborhydrid und einem Ueberschuss von Eisessig herstellen.

Erfindungsgemäss verwendbare saure Reagenzien sind starke und schwache Säuren des Brönstedt Typs, beispielsweise Mineralsäuren, z.B. Schwefelsäure oder Salzsäure, Borsäure, organische Säuren oder Lewis-Säuren gegebenenfalls im Gemisch mit organischen Säuren.

Erfindungsgemäss verwendbare organische Säuren sind Carbonsäuren, Dicarbonsäuren oder Sulfonsäure. Geeignete organische Säuren sind insbesondere aliphatische, cycloaliphatische oder aromatische Carbonsäuren,

Dicarbonsäuren oder Sulfonsäuren, die gegebenenfalls durch Halogen, z.B. Chlor, Niederalkoxy, z.B. Methoxy, Hydroxy oder durch Aryl, z.B. Phenyl, substituiert sind.

Aliphatische Carbonsäuren sind beispielsweise gegebenenfalls wie oben angegeben substituierte Niederalkancarbonsäuren, z.B. Ameisen-, Essig-, Propion-, Butter-, Chloressig-, Trifluoressig- oder Phenylessigsäure. Cycloaliphatische Carbonsäuren sind beispielsweise gegebenenfalls wie oben angegeben substituierte Cyclopentan- oder Cyclohexancarbonsäuren. Aromatische Carbonsäuren sind beispielsweise Benzoesäure oder wie oben angegeben substituierte Benzoesäure, z.B. Chlorbenzoesäure oder Methoxybenzoesäure.

Aliphatische Dicarbonsäuren sind beispielsweise gegebenenfalls wie oben angegeben substituierte Niederalkandicarbonsäuren, z.B. Oxal-, Malon-, Bernstein-, Glutar- oder Adipinsäure.

Aliphatische Sulfonsäuren sind beispielsweise Methansulfonsäure oder Aethansulfonsäure. Aromatische Sulfonsäuren sind beispielsweise gegebenenfalls wie oben angegeben substituierte Arylsulfonsäuren, z.B. Benzolsulfonsäure oder p-Toluolsulfonsäure.

Erfindungsgemäss verwendbare Lewis-Säuren, welche gegebenenfalls im Gemisch mit den vorstehend genannten organischen Säuren eingesetzt werden können, wirken nicht oxydierend oder reduzierend und sind beispielsweise Uebergangsmetallhalogenide, z.B. Titan-(IV)-chlorid, Zirkon-(IV)-chlorid, Nickelchlorid, Zinkchlorid, Eisen-(III)-chlorid, Kobalt-(II)-chlorid oder Chrom-(III)-chlorid, oder Halogenide von Elementen der 3. und 4. Hauptgruppe des Periodensystems, z.B. Aluminiumtrichlorid, Siliciumtetrachlorid oder Zinntetrachlorid.

Bevorzugte saure Reagenzien sind beispielsweise Essigsäure, Ameisensäure, sowie Kobalt-(II)-chlorid oder Titantetrachlorid im Gemisch mit Essigsäure.

Die genannten sauren Reagenzien werden in Mengen von mindestens einem Mol pro Mol eingesetzter Verbindung der Formel II verwendet, bevorzugt in grossem Ueberschuss, gegebenenfalls als Lösungsmittel. Mineralsäuren gelangen in verdünnter Form, z.B. als methanolische Lösung, zur Anwendung.

Die erfindungsgemässe Reduktion wird entweder in einer der obengenannten organischen flüssigen Säuren allein oder unter Zusatz eines weiteren Lösungsmittels durchgeführt. Als zusätzliche Lösungsmittel geeignet sind inerte, die Reduktion nicht störende, gegebenenfalls halogenierte Kohlenwasserstoffe, z.B. Benzol, Toluol, Xylol, Methylenchlorid, Aether, z.B. Diäthyläther, Niederalkylenglycoldiniederalkyläther, z.B. Dimethoxyäthan oder Diäthylenglykoldimethyläther, cyclische Aether, z.B. Dioxan oder Tetrahydrofuran, Carbonsäureamide, z.B. Dimethylformamid oder Niederalkanole, z.B. Methanol, Aethanol oder tert.-Butanol, oder Gemische davon. Falls notwendig, wird in einer Inertgas-, z.B. Argon- oder Stickstoffatmosphäre, gearbeitet.

Die Reaktionstemperatur liegt zwischen ca. -20° und ca. +80° und bevorzugt zwischen ca. 0° und ca. 30°.

Bevorzugt setzt man eine Verbindung der Formel II, z.B. 7β-Phenoxyacetylamino-3-morpholino-3-cephem-4-carbonsäurebenzhydrylester, 7β-Phenoxyacetylamino-3-piperidino-3-cephem-4-carbonsäurebenzhydrylester, 7β-Benzylacetylamino-3-morpholino-3-cephem-4-carbonsäurebenzhydrylester, 7β-[2-(2-Thienyl)-acetylamino]-3-pyrrolidino-3-cephem-4-carbonsäure-p-nitrobenzylester-1-oxid oder 7β-Phenoxyacetylamino-3-pyrrolidino-3-cephem-4-carbonsäurebenzhydrylester mit Natrium- oder Kaliumborhydrid, Natriumcyanborhydrid und Natriumtriacetyloxyborhydrid als komplexen Borhydriden in Gegenwart von Eisessig oder von Kobalt(II)-chlorid oder Titan(IV)-chlorid in Eisessig als saure Reagenzien und Dimethylformamid, Tetrahydrofuran, Aethanol, Methylenchlorid, Xylol, Toluol oder Aethylenglycoldimethyläther als Lösungsmittel bei Temperaturen von 0° bis ca. 30° um.

Erfindungsgemäss erhältliche Verbindungen der Formel I, worin $R_1^a$ eine Aminoschutzgruppe $R_1^A$, $R_1^b$ Wasserstoff oder eine Acylgruppe Ac, $R_1^a$ und $R_1^b$ zusammen eine bivalente Aminoschutzgruppe und $R_2$ einen zusammen mit der Carbonylgruppierung eine geschützte Carboxylgruppe bildenden Rest darstellen,können durch Desacylierung und Abspaltung der 4-Carboxylschutzgruppe analog zu den in den Deutschen Offenlegungsschriften 1.795.868 und 1.445.615 beschriebenen Verfahren in Verbindungen der Formel I umgewandelt werden, worin $R_1^a$ und $R_1^b$ Wasserstoff und $R_2$ Hydroxy bedeuten.

Erfindungsgemäss erhältliche Verbindungen der Formel I, worin $R_1^a$ und $R_1^b$ Wasserstoff bedeuten und $R_2$ für Hydroxy oder einen zusammen mit der Carbonylgruppierung -C(=O)- eine geschützte Carboxylgruppe bildenden Rest steht, können analog zu den in der Deutschen Offenlegungsschrift 2.151.567 und den publizierten europäischen Patentanmeldungen Nr. 0008343, 0007470 und 0013762 beschriebenen Verfahren durch Acylierungsreaktion der 7β-Aminogruppe in Verbindungen der Formel I umgewandelt werden, worin $R_1^a$ eine Aminoschutzgruppe $R_1^A$ und $R_1^b$ Wasserstoff oder eine Acylgruppe Ac oder $R_1^a$ und $R_1^b$ zusammen eine bivalente Aminoschutzgruppe darstellen.

Salze von Verbindungen der Formel I können in an sich bekannter Weise hergestellt werden. So kann man Salze von solchen Verbindungen mit sauren Gruppen z.B. durch Behandeln mit Metallverbindungen, wie Alkalimetallsalzen von geeigneten Carbonsäuren, z.B. dem Natriumsalz der α-Aethylcapronsäure, oder mit Ammoniak oder einem geeigneten organischen Amin bilden, wobei man vorzugsweise stöchiometrische

Mengen oder nur einen kleinen Ueberschuss des salzbildenden Mittels verwendet. Säureadditionssalze von Verbindungen der Formel I mit basischen Gruppierungen erhält man in üblicher Weise, z.B. durch Behandeln mit einer Säure oder einem geeigneten Anionenaustauschreagens. Innere Salze von Verbindungen der Formel I, welche z.B. eine salzbildende Aminogruppe und eine freie Carboxylgruppe enthalten, können z.B. durch Neutralisieren von Salzen, wie Säureadditionssalzen, auf den isoelektrischen Punkt, z.B. mit schwachen Basen, oder durch Behandeln mit flüssigen Ionenaustauschern gebildet werden. Salze von 1-Oxyden von Verbindungen der Formel II mit salzbildenden Gruppen können in analoger Weise hergestellt werden.

Salze können in üblicher Weise in die freien Verbindungen übergeführt werden, Metall- und Ammoniumsalze z.B. durch Behandeln mit geeigneten Säuren, und Säureadditionssalze z.B. durch Behandeln mit einem geeigneten basischen Mittel.

Erhaltene Gemische von Isomeren können nach an sich bekannten Methoden, in die einzelnen Isomeren getrennt werden, Gemische von diastereomeren Isomeren z.B. durch fraktioniertes Kristallisieren, Absorptionschromatographie (Kolonnen- oder Dünnschichtchromatographie) oder andere geeignete Trennverfahren. Erhaltene Racemate können in üblicher Weise, gegebenenfalls nach Einführen von geeigneten salzbildenden Gruppierungen, z.B. durch Bilden eines Gemisches von diastereoisomeren Salzen mit optisch aktiven salzbildenden Mitteln, Trennen des Gemisches in die diastereoisomeren Salze und Verwandlung der Salze in die freien Verbindungen oder durch fraktioniertes Kristallisieren aus optisch aktiven Lösungsmitteln, in die Antipoden getrennt werden.

Das Verfahren umfasst auch diejenigen Ausführungsformen, wonach als Zwischenprodukte anfallende Verbindungen als Ausgangsstoffe verwendet und die restlichen Verfahrensschritte mit diesen durchgeführt werden,

oder das Verfahren auf irgendeiner Stufe abgebrochen wird; ferner können Ausgangsstoffe in Form von Derivaten verwendet oder während der Reaktion gebildet werden.

Vorzugsweise werden solche Ausgangsstoffe verwendet und die Reaktionsbedingungen so gewählt, dass man zu den eingangs als besonders bevorzugt aufgeführten Verbindungen gelangt.

Verbindungen der Formel II, worin der Index n den Wert 1 hat, sind neu und ebenfalls Gegenstand der vorliegenden Erfindung. Sie werden erhalten, indem man eine Verbindung der Formel

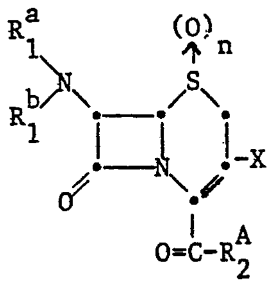

(III),

worin der Index n den Wert 1 und X Hydroxy, Chlor, Brom oder eine Sulfonyloxygruppe der Formel $-O-SO_2-R_4$ bedeutet und $R_1^a$, $R_1^b$ und $R_2$ die unter Formel I genannten Bedeutungen haben und worin, falls X Hydroxy bedeutet, $R_2$ nicht Hydroxy sein kann, mit einem primären oder sekundären Amin der Formel $H-N(R_3^a)(R_3^b)$ umsetzt und, wenn erwünscht, ein erhältliches Salz in die freie Verbindung oder in ein anderes Salz und/oder eine erhältliche freie Verbindung mit einer salzbildenden Gruppe in ein Salz überführt.

In einer Sulfonyloxygruppe der Formel $-O-SO_2-R_4$ ist $R_4$ ein gegebenenfalls substituierter, insbesondere aliphatischer, cycloaliphatischer, araliphatischer oder aromatischer Kohlenwasserstoffrest mit bis zu 18, vorzugsweise bis zu 10, Kohlenstoffatomen. Geeignete Gruppen $R_4$ sind beispielsweise gegebenenfalls substituierte, wie durch Niederalkoxy, wie Methoxy, Halogen, wie Fluor, Chlor oder Brom, Aryl, wie Phenyl,

Aryloxy, wie Phenyloxy, mono- oder polysubstituierte Alkyl-, insbesondere Niederalkyl-, wie Methyl-, Aethyl- oder Butylgruppen, Alkenyl-, wie Allyl- oder Butenylgruppen, Cycloalkyl-, wie Cyclopentyl- oder Cyclohexylgruppen, oder gegebenenfalls durch Niederalkyl, wie Methyl, Niederalkoxy, wie Methoxy, Halogen, wie Fluor, Chlor oder Brom, Aryl, wie Phenyl, Aryloxy, wie Phenyloxy, oder Nitro, mono- oder polysubstituierte Naphthyl- oder insbesondere Phenylgruppen, beispielsweise Phenyl, o-, m- oder bevorzugt p-Tolyl, o-, m- oder bevorzugt p-Methoxyphenyl, o-, m- oder p-Chlorphenyl, p-Biphenylyl, p-Phenoxyphenyl, p-Nitrophenyl oder 1- oder 2-Naphthyl.

Die Umsetzung wird bevorzugt in einem geeigneten inerten polaren Lösungsmittel durchgeführt. Solche geeigneten Lösungsmittel sind beispielsweise gegebenenfalls halogenierte Kohlenwasserstoffe, z.B. Benzol, Toluol, Xylol oder Methylenchlorid, Aether, z.B. Diäthyläther, Niederalkylenglycoldiniederalkyläther, z.B. Dimethoxyäthan oder Diäthylenglykoldimethyläther, cyclische Aether, z.B. Dioxan oder Tetrahydrofuran, Carbonsäureamide, z.B. Dimethylformamid oder Niederalkanole, z.B. Methanol, Aethanol oder tert.-Butanol, oder Gemische davon. Falls notwendig wird in einer Inertgas-, z.B. Argon- oder Stickstoffatmosphäre, gearbeitet.

Die Reaktionstemperatur liegt zwischen ca. -20° und ca. +80° und bevorzugt zwischen ca. 0° und ca. 30°. Das primäre oder sekundäre Amin wird, bezogen auf das Ausgangsmaterial der Formel III, in mindestens äquivalenter Menge, bevorzugt in einer Menge von 2-5 Aequivalenten zugesetzt.

Die Ausgangsverbindungen der Formel III sind bekannt oder können auf an sich bekannte Weise, z.B. analog den Deutschen Offenlegungsschriften 2.331.148, 2.506.330 oder 2.606.196 hergestellt werden.

Verbindungen der Formel II, worin der Index n den Wert 0 hat, sind bekannt oder können auf an sich bekannte Weise, z.B. analog der Deutschen Offenlegungsschrift 2.606.196, hergestellt werden.

Verbindungen der Formel II können aus Verbindungen der Formel III oder gemäss dem in der Deutschen Offenlegungsschrift 2.606.196 beschriebenen Verfahren in situ hergestellt und ohne Isolierung zu Verbindungen der Formel I weiterverarbeitet werden.

Die folgenden Beispiele dienen zur Illustration der Erfindung ohne sie einzuschränken. Temperaturen werden in Celsiusgraden angegeben.

Beispiel 1: Man löst 5,86 g (10 mMol) 7β-Phenoxyacetylamino-3-morpholino-3-cephem-4-carbonsäurebenzhydrylester in 25 ml Dimethylformamid und 25 ml Eisessig, kühlt auf 0° ab und gibt eine Stunde lang in Portionen von 100 mg 2,04 g Natriumborhydrid zu, wobei jedesmal beträchtliche Gasentwicklung auftritt. Man rührt weitere 5 Stunden bei Raumtemperatur. Anschliessend verdünnt man mit 300 ml Eiswasser und extrahiert 4 mal mit jeweils 100 ml Methylenchlorid. Nach Neutralisieren mit wässriger Natriumhydrogencarbonatlösung wird die organische Phase im Vakuum eingeengt. Das Dimethylformamid wird im Hochvakuum abdestilliert. Der ölige, orangefarbene Rückstand wird mit Toluol/Essigester (95:5) an Kieselgel chromatographiert und ergibt reinen 7β-Phenoxyacetylamino-3-cephem-4-carbonsäurebenzhydrylester als weissen Schaum.

Beispiel 2: Zu einer Lösung von 5,86 g (10 mMol) 7β-Phenoxyacetylamino-3-morpholino-3-cephem-4-carbonsäurebenzhydrylester in 50 ml Methylenchlorid und 50 ml Eisessig tropft man innerhalb von 20 min eine Lösung von 1,05 g Natriumcyanborhydrid in 30 ml Tetrahydrofuran. Es tritt beträchtliche Gasentwicklung auf. Nach Zugabe von 20 ml Ameisensäure rührt man 16 Stunden bei Raumtemperatur. Zur Aufarbeitung verdünnt

man das Reaktionsgemisch mit 300 ml Methylenchlorid und wäscht die organische Phase 4 mal mit 100 ml Wasser, trocknet über Natriumsulfat und destilliert das Lösungsmittel im Vakuum ab. Das nahezu farblose Rohprodukt wird mit Toluol/Essigester (9:1) an Kieselgel chromatographiert und ergibt reinen 7β-Phenoxyacetylamino-3-cephem-4-carbonsäurebenzhydrylester.

Beispiel 3: Zu einer Lösung von 11,7 g (20 mMol) 7β-Phenoxyacetalamino-3-piperidino-3-cephem-4-carbonsäurebenzhydrylester in 50 ml Dimethylformamid gibt man in mehreren Portionen bei 0° bis 5° 4,76 g (20 mMol) Kobalt(II)-chloridhexahydrat zu. Die Lösung färbt sich dunkelblau. Bei einer Innentemperatur von 0° tropft man 75 ml Eisessig innerhalb von 30 min zu. Anschliessend gibt man innerhalb von 1 Stunde 4,08 g (120 mMol) Natriumborhydrid in Portionen von 200 mg zu, wobei jeweils heftige Gasentwicklung einsetzt. Nach beendeter Zugabe lässt man auf Raumtemperatur erwärmen und rührt nach Zugabe von weiteren 50 ml Eisessig noch 36 Stunden bei Raumtemperatur. Die dunkelrot gefärbte Lösung wird vom rosarot gefärbten Niederschlag abfiltriert, der Filterrückstand mit Methylenchlorid nachgewaschen und das Filtrat im Vakuum eingeengt. Das Dimethylformamid wird im Hochvakuum abdestilliert. Der ölige Rückstand wird in Methylenchlorid gelöst und dreimal mit 50 ml Wasser gewaschen. Die Methylenchloridlösung wird über 50 g Kieselgel filtriert, um restliche Kobaltsalze zu beseitigen. Das Eluat wird eingedampft, umd man erhält reinen 7β-Phenoxyacetylamino-3-cephem-4-carbonsäurebenzhydrylester als weissen Schaum.

Beispiel 4: Man löst 11,4 g (20 mMol) 7β-Benzylacetylamino-3-morpholino-3-cephem-4-carbonsäurebenzyhydrylester in 50 ml Aethanol, 50 ml Methylenchlorid und 50 ml Eisessig. Die Lösung wird auf eine Innentemperatur von 0° abgekühlt. Unter Rühren und Stickstoffatmosphäre werden dann innerhalb einer Stunde 4,08 g (120 mMol) Natriumborhydrid in Portionen von 200 mg eingetragen, wobei jeweils starke Gasentwicklung zu beobachten ist. Nach beendeter Zugabe wird auf Raumtemperatur

erwärmt, mit weiteren 50 ml Eisessig versetzt und anschliessend 36 Stunden bei Raumtemperatur gerührt. Die klare, nahezu farblose Lösung wird am Rotationsverdampfer eingeengt und der Rückstand zwischen Wasser und Methylenchlorid verteilt. Die organische Phase wird nochmals mit 2 mal 50 ml Wasser gewaschen und anschliessend im Vakuum bis zum Schaum eingeengt. Man erhält rohen 7β-Benzylacetylamino-3-cephem-4-carbonsäurebenzhydrylester.

Beispiel 5: a) Zu einer Mischung von 9,5 g (50 mMol) Titan(IV)-chlorid und 5,67 g (150 mMol) Natriumborhydrid in 150 ml Dimethoxyäthan gibt man bei -20° langsam eine Suspension von 10,86 g (20 mMol) 7β-[2-(2-Thienyl)-acetylamino]-3-pyrrolidino-3-cephem-4-carbonsäure-p-nitrobenzylester-1-oxid in 150 ml Aethylenglycoldimethyläther so zu, dass die Innentemperatur nicht über -15° ansteigt. Nach beendeter Zugabe lässt man auf 0°C erwärmen und tropft dann eine Mischung aus 50 ml Eisessig und 50 ml Aethylenglycoldimethyläther zu. Anschliessend rührt man weitere 36 Stunden bei Raumtemperatur. Zur blau gefärbten Reaktionslösung tropft man vorsichtig unter Eiskühlung 200 ml Eiswasser und extrahiert mit 3 mal 150 ml Methylenchlorid. Nach Trocknen über Natriumsulfat und Abdestillieren des Lösungsmittels im Vakuum erhält man nahezu reinen 7β-[2-(2-Thienyl)-acetylamino]-3-cephem-4-carbonsäure-p-nitrobenzylester als Schaum.

b) Das Ausgangsmaterial kann wie folgt hergestellt werden:
Zu einer Lösung von 19,6 g (40 mMol) 7β-[2-(2-Thienyl)-acetylamino]-3-hydroxy-3-cephem-4-carbonsäure-p-nitrobenzylester-1-oxid in 150 ml Methylenchlorid gibt man bei -10° 9,2 g (80 mMol) Methansulfonsäurechlorid. Anschliessend tropft man innerhalb einer Stunde bei -10° 8,1 g (80 mMol) Triäthylamin zu und rührt eine weitere Stunde bei dieser Temperatur. Danach tropft man innerhalb einer Stunde bei -10° 8,53 g (120 mMol) Pyrrolidin zu und lässt nach beendeter Zugabe auf 25° erwärmen. Die organische Phase wird mit 3 mal 100 ml Wasser gewaschen und im Vakuum eingeengt. Der Rückstand wird aus 200 ml Methanol kristallisiert. Man erhält reines 7β-[2-(2-Thienyl-acetylamino]-3-pyrrolidino-3-cephem-4-carbonsäure-p-nitrobenzylester-1-oxid.

Beispiel 6: Zur Lösung von 11,4 g (20 mMol) 7β-Phenoxyacetylamino-3-pyrrolidino-3-cephem-4-carbonsäurebenzhydrylester in 50 ml Methylenchlorid, 50 ml Aethanol und 50 ml Eisessig gibt man unter Eiskühlung eine Lösung von 5,4 g (100 mMol) Kaliumborhydrid in 50 ml Dimethylformamid innerhalb einer Stunde zu. Danach rührt man 36 Stunden bei Raumtemperatur, engt auf das halbe Volumen ein, versetzt mit 300 ml Eiswasser und extrahiert mehrfach mit Methylenchlorid. Nach Abdestillieren des Lösungsmittels im Vakuum erhält man 7β-Phenoxyacetylamino-3-cephem-4-carbonsäurebenzhydrylester als weissen Schaum.

Beispiel 7:

a) Man stellt eine Suspension aus 142 g (3,75 mMol) Natriumborhydrid und 2,4 l abs. Toluol her und gibt innerhalb von 4 Stunden 2,2 l Eisessig bei einer Innentemperatur von 20° hinzu. Man leitet den bei dieser exothermen Reaktion gebildeten Wasserstoff ab.
Zu dem so gebildeten Triacetoxyborhydrid lässt man anschliessend bei 20° 400 g (0.683 mMol) 7β-Phenoxyacetylamino-3-morpholino-3-cephem-4-carbonsäurebenzhydrylester in 1,2 l Eisessig und 0,6 l abs. Toluol zulaufen. Man stellt die Temperatur des Reaktionsgemisches auf eine Innentemperatur von 40° ein und rührt bei dieser Temperatur 24 Stunden lang. Danach destilliert man das Lösungsmittel im Vakuum vollständig ab und verteilt den öligen Rückstand zwischen 1,4 l Wasser und 1,4 l Toluol. Nach Abtrennung der Phasen extrahiert man die wässrige Phase nochmals mit 0,6 l Toluol. Anschliessend neutralisiert man die gesammelten organischen Phasen mit gesättigter Natriumhydrogencarbonatlösung und wäscht zweimal mit 500 ml Wasser. Man dampft die organische Phase bei reduziertem Druck ein, löst das erhaltene Oel in 1,5 l Aethanol bei 55°, woraus unter langsamem Abkühlen und Animpfen

7β-Phenoxyacetylamino-3-cephem-4-carbonsäurebenzhydrylester kristallisiert.

b) Zu einer Lösung von 20 g (40 mMol) 7β-Phenoxyacetylamino-3-cephem-4-carbonsäurebenzhydrylester in 400 ml abs. Methylenchlorid gibt man bei -15° 38,6 ml (0,48 mMol) abs. Pyridin und 320 ml einer 8-prozentigen Lösung (0,12 mMol) Phosphorpentachlorid in abs. Methylenchlorid. Man rührt jeweils 30 Minuten bei -10° und -5°, kühlt die gelbe Lösung auf -20° ab und gibt danach 270 ml abs. Methanol hinzu. Die Innentemperatur soll nicht über -10° ansteigen. Nach weiterem Rühren von jeweils 1 Stunde bei -10° und dann bei Raumtemperatur stellt man langsam durch Zugabe von etwas Kaliumdihydrogenphosphatlösung und einigen Tropfen Phosphorsäure den pH auf 2.0 ein. Man trennt die wässrige Phase ab und extrahiert zweimal mit je 100 ml Methylenchlorid. Man vereinigt die organischen Phasen, wäscht zweimal mit je 50 ml Wasser und trocknet über Natriumsulfat. Nach Abdestillieren des Lösungsmittels löst man den öligen Rückstand in 20 ml Essigsäureäthylester und versetzt mit 11 g p-Toluolsulfonsäuremonohydrat, gelöst in 150 ml Essigsäureäthylester. Man dampft ein und löst den Rückstand in Methylchlorid auf, worauf mit Aether 7β-Amino-3-cephem-4-carbonsäurebenzhydrylester-p-toluolsulfonat als weisser Niederschlag ausfällt.

c) Man setzt 36.6 g 7β-Amino-3-cephem-4-carbonsäurebenzhydrylester in 60 ml Anisol mit 240 ml Trifluoressigsäure 10 Minuten bei Raumtemperatur um. Anschliessend verdünnt man mit Toluol und dampft im Vakuum vollständig ein. Man löst den Rückstand in einem Gemisch aus 150 ml Methanol, 250 ml Aether und 10 ml Wasser und stellt mit Triäthylamin auf pH 3.5. Man kühlt die entstandene Suspension während 1 Stunde auf 0°C ab und filtriert den feinkristallinen Niederschlag von 7β-Amino-3-cephem-4-carbonsäure.

d) Man legt 4,23 ml (5.5 mMol) Dimethylformamid vor und tropft innerhalb von 5 Minuten 5.02 ml (5.5 mMol) Phosphoroxychlorid so zu, dass die Innentemperatur nicht über 40° steigt. Nach beendeter Zugabe wird noch 30 Minuten bei einer Innentemperatur von 40° gerührt. Man kühlt auf 20° ab und verdünnt mit 60 ml Essigsäureäthylester. Man kühlt auf 0° ab und versetzt mit 11,46 g (5 mMol) 2-(2-Formyl-amino-4-thiazolyl)-2-methoxyiminoessigsäure, wobei die Temperatur auf 8°C ansteigt. Anschliessend rührt man die entstehende klare, hellgelbe Lösung noch 2 Stunden bei 0°. In einem zweiten Reaktionsgefäss wird 10.0 g (5 mMol) 7β-Amino-3-cephem-4-carbonsäure in 60 ml Essigester suspendiert und mit 15.32 ml (6.25 mMol) N,O-Bistrimethyl-silylacetamid versetzt und 30 Minuten bei Raumtemperatur gerührt. Man kühlt auf -36° ab und gibt die Lösung mit dem Säurechlorid der 2-(2-Formylamino-4-thiazolyl)-2-methoxyiminoessigsäure innerhalb 30 Minuten zu, wobei die Innentemperatur auf -3° ansteigt. Man rührt die entstehende gelbe, klare Lösung 45 Minuten bei -10° und giesst diese dann auf 400 ml gesättigte Sodalösung und stellt durch Zugabe weiterer Sodalösungen den pH-Wert auf 7.8 ein.
Man trennt die Phasen, extrahiert die wässrige Phase mit 200 ml Essigester und wäscht die nachextrahierte organische Phase mit 10 ml Wasser. Man stellt die wässrige Phase mit 30 ml konz. Salzsäure auf pH 1.7 und extrahiert mit 3 x 300 ml Essigsäureäthylester. Man wäscht die vereinigten organischen Phasen mit 2 x 100 ml gesättigter Kochsalzlösung und trocknet über Natriumsulfat. Man engt die organische Phase auf ca. 400 ml ein, woraus über Nacht 7β-[2-(2-Amino-4-thiazolyl)-2-methoxyiminoacetamido]-3-cephem-4-carbonsäure auskristallisiert.

e) Eine Mischung von 2,25 ml Chlormethylpivalat und 9,0 g Natriumjodid in 30 ml Aceton wird 3 Stunden bei Raumtemperatur gerührt. Das Gemisch wird mit einer Lösung von 2,6 g 7β-[2-(2-tert.Butoxy-carbonylamino-4-thiazolyl)-2-methoxyiminoacetamido]-3-cephem-4-carbonsäure-natriumsalz in 50 ml Dimethylformamid versetzt und 1 Stunde bei Raumtemperatur weitergerührt. Nach Einengen im Vakuum wird der Rückstand in Aethylacetat gelöst und die Lösung mit gesättigter wässriger Natriumchloridlösung gewaschen und über Natriumsulfat getrocknet. Das nach Eindampfen im Vakuum erhaltene Rohprodukt wird an Kieselgel chromatographiert. Elution mit Toluol enthaltend 20-30% Aethylacetat ergibt den 7β-[2-(2-tert.Butoxycarbonylamino-4-thiazolyl)-2-methoxyiminoacetamido]-3-cephem-4-carbonsäure-pivaloyloxymethylester. Dieser wird in 8,7 ml Methylenchlorid mit 8,7 ml Trifluoressigsäure versetzt und 1 Stunde bei Raumtemperatur gerührt. Nach Zugabe von kaltem Toluol wird im Vakuum eingedampft. Der Rückstand wird mit Diäthyläther/Hexan 1:1 verrührt, abfiltriert und getrocknet. Das erhaltene braune Pulver wird in Aethylacetat aufgenommen, mit kalter gesättigter Natriumhydrogencarbonat- und gesättigter wässriger Natriumchlorid-Lösung gewaschen, über Natriumsulfat getrocknet und im Vakuum eingedampft. Der Rückstand wird mit Diäthyläther digeriert, abfiltriert und im Hochvakuum getrocknet. Der erhaltene 7β-[2-(2-Amino-4-thiazolyl)-2-methoxyiminoacetamido]-3-cephem-4-carbonsäure-pivaloyloxymethylester zersetzt sich ab etwa 130°C.

Beispiel 8: Analog den in den Beispielen 1-7 beschriebenen Verfahren können, ausgehend von den entsprechenden Verbindungen der Formel II und gegebenenfalls nach Desacylierungs- und Acylierungsreaktionen an der 7β-Aminogruppe, folgende Verbindungen der Formel I erhalten werden:

7β-Phenyloxyacetylamino-3-cephem-4-carbonsäure,
7β-Phenylacetylamino-3-cephem-4-carbonsäure,
7β-Thien-2- oder 3-ylacetylamino-3-cephem-4-carbonsäure,
7β-Fur-2- oder 3-ylacetylamino-3-cephem-4-carbonsäure,
7β-(5-Carboxyl-5-aminovalerylamino)-3-cephem-4-carbonsäure und die tertiär-Butyl-, Trichloräthyl-, Benzyl-, p-Methoxybenzyl-, p-Nitrobenzyl- und Diphenylmethylester von diesen Verbindungen;

7β-[D-2-Amino-2-phenylacetylamino]-3-cephem-4-carbonsäure,
7β-[D-2-t.Butyloxycarbonylamino-2-phenylacetylamino]-3-cephem-4-carbonsäure-t.butylester,
7β-[D-2-Amino-2-(1,4-cyclohexadienyl)-acetylamino]-3-cephem-4-carbonsäure,
sowie der Trichloräthyl-, Benzyl-, p-Methoxyphenyl-, p-Nitrophenyl- und Diphenylmethylester;
7β-[D-2-t.Butyloxycarbonylamino-2-(1,4-cyclohexadienyl)-acetylamino]-3-cephem-4-carbonsäure-t.butylester,
sowie der Trichloräthyl-, Benzyl-, p-Methoxyphenyl-, p-Nitrobenzyl- und Diphenylmethylester;
7β-[2-(2-Amino-4-thiazolyl)-2-methoxyimino-acetylamino]-3-cephem-4-carbonsäure,
7β-[2-(2-t.Butyloxycarbonylamino-4-thiazolyl)-2-methoxyimino-acetylamino]-3-cephem-4-carbonsäure-t.butylester,
sowie der Trichloräthyl-, Benzyl-, p-Methoxyphenyl-, p-Nitrobenzyl- und Diphenylmethylester;
7β-[2-(5-Amino-1,2,4-thiadiazol-3-yl)-2-methoxyimino-acetylamino]-3-cephem-4-carbonsäure,
7β-[2-(5-t.Butyloxycarbonylamino-1,2,4-thiadiazol-3-yl)-2-methoxyimino-acetylamino]-3-cephem-4-carbonsäure-t.butylester,
sowie der Trichloräthyl-, Benzyl-, p-Methoxyphenyl-, p-Nitrobenzyl- und Diphenylmethylester;
7β-[2-(5-Amino-1,2,4-thiadiazol-3-yl)-2-(carboxyprop-2-yloxyimino)-acetylamino]-3-cephem-4-carbonsäure,
7β-[2-(5-t.Butyloxycarbonylamino-1,2,4-thiadiazol-3-yl)-2-(carboxyprop-2-yloxyimino)-acetylamino]-3-cephem-4-carbonsäure-t.butylester,
sowie der Trichloräthyl, Benzyl-, p-Methoxyphenyl-, p-Nitrobenzyl- und Diphenylmethylester;
7β-[2-(5-Amino-1,2,4-thiadiazol-3-yl)-2-hydroxyimino-acetylamino]-3-cephem-4-carbonsäure,
7β-[2-(5-t.Butyloxycarbonylamino-1,2,4-thiadiazol-3-yl)-2-hydroxyimino-acetylamino]-3-cephem-4-carbonsäure-t.butylester,

sowie der Trichloräthyl-, Benzyl-, p-Methoxyphenyl-, p-Nitrobenzyl- und Diphenylmethylester;

7β-[2-(5-N-Methylamino-1,2,4-thiadiazol-3-yl)-2-Z-methoxyimino-acetylamino]-3-cephem-4-carbonsäure,

sowie der t.Butyl-, Trichloräthyl-, Benzyl-, p-Methoxyphenyl-, p-Nitrobenzyl- und Diphenylmethylester;

7β-Amino-3-cephem-4-carbonsäure,

7β-Amino-3-cephem-4-carbonsäure-tert.butylester,

7β-Amino-3-cephem-4-carbonsäure-trichloräthylester,

7β-Amino-3-cephem-4-carbonsäure-benzylester,

7β-Amino-3-cephem-4-carbonsäure-p-methoxybenzylester,

7β-Amino-3-cephem-4-carbonsäure-p-nitrobenzylester und

7β-Amino-3-cephem-4-carbonsäurediphenylmethylester.

In den obigen Verbindungen können die Aminogruppen in den 7β-Acylresten ebenfalls durch die Trichloräthoxycarbonylgruppe oder Chloracetylgruppe geschützt sein.

Patentansprüche

1. Verfahren zur Herstellung von 7β-Amino-3-cephem-4-carbonsäureverbindungen der Formel

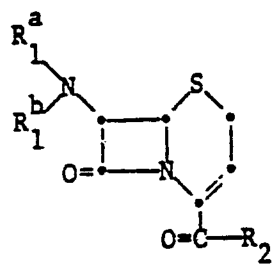

(I),

worin $R_1^a$ Wasserstoff oder eine Aminoschutzgruppe $R_1^A$, $R_1^b$ Wasserstoff oder eine Acylgruppe Ac oder $R_1^a$ und $R_1^b$ zusammen eine bivalente Aminoschutzgruppe und $R_2$ Hydroxy oder einen zusammen mit der Carbonylgruppierung -C(=O)- eine geschütze Carboxylgruppe bildenden Rest darstellen, sowie Salzen von Verbindungen der Formel I mit salzbildenden Gruppen, dadurch gekennzeichnet, dass man eine Verbindung der Formel

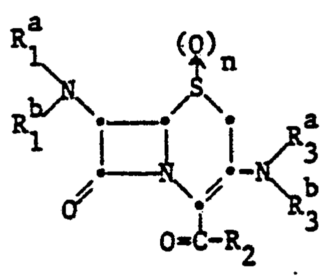

(II),

worin der Index n den Wert 0 oder 1 hat, $R_1^a$, $R_1^b$ und $R_2$ die unter Formel I genannten Bedeutungen haben und die Gruppe der Formel $-N(R_3^a)(R_3^b)$ für eine sekundäre oder tertiäre Aminogruppe steht, mit einem geeigneten Borhydrid in Gegenwart eines sauren Reagenzes umsetzt und in an sich bekannter Weise, wenn erwünscht, eine erfindungsgemäss erhältliche Verbindung der Formel I in eine andere Verbindung der Formel I umwandelt, und/oder in eine Verbindung der Formel I in geschützter Form vorliegende funktionelle Gruppen in die freien funktionellen Gruppen überführt und/oder ein erhältliches Salz in

die freie Verbindung oder in ein anderes Salz überführt, und/oder eine erhältliche freie Verbindung mit einer salzbildenden Gruppe in ein Salz überführt und/oder ein erhältliches Gemisch von isomeren Verbindungen der Formel I in die einzelnen Isomeren auftrennt.

2. Verfahren nach Patentanspruch 1 zur Herstellung von Verbindungen der Formel I, dadurch gekennzeichnet, dass $R_1^a$ Wasserstoff oder eine Acylgruppe Ac, welche in einem fermentativ oder bis-, halb-, oder totalsynthetisch herstellbaren N-Acylderivat eine 6β-Amino-penam-3-carbonsäure- oder 7β-Amino-3-cephem-4-carbonsäureverbindung enthalten ist, $R_1^b$ Wasserstoff bedeutet und $R_2$ die in Anspruch 1 genannten Bedeutungen hat.

3. Verfahren nach Patentanspruch 2 zur Herstellung von Verbindungen der Formel I, dadurch gekennzeichnet, dass eine Acylgruppe Ac eine Gruppe der Formel

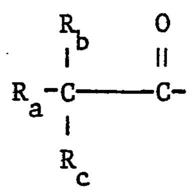 (IA),

bedeutet, worin (1) $R_a$ einen unsubstituierten oder substituierten carbocyclischen Arylrest, z.B. Phenyl, einen unsubstituierten oder substituierten, vorzugsweise ungesättigten, cycloaliphatischen Kohlenwasserstoffrest, z.B. Cyclohexadienyl oder Cyclohexenyl, oder einen unsubstituierten oder substituierten heterocyclischen Arylrest, z.B. Thienyl, Furyl, Thiazolyl oder Thiadiazolyl, $R_b$ Wasserstoff und $R_c$ Wasserstoff oder unsubstituiertes oder substituiertes, insbesondere geschütztes, Hydroxy, Amino, Carboxyl oder Sulfo darstellen, oder worin (2) $R_a$ freies oder geschütztes ω-Amino-ω-carboxyniederalkyl, z.B. -propyl, Cyan, veräthertes Hydroxy oder Mercapto, z.B. unsubstituiertes oder substituiertes Phenyloxy, Phenylthio oder Pyridylthio, oder einen unsubstituierten oder substituierten, über ein

Ringstickstoffatom verknüpften, ungesättigten heterocyclischen Rest, z.B. Tetrazolyl, und $R_b$ und $R_c$ Wasserstoff bedeuten, oder worin (3) $R_a$ einen unsubstituierten oder substituierten carbocyclischen Arylrest, z.B. Phenyl, oder einen unsubstituierten oder substituierten heterocyclischen Arylrest, z.B. Thienyl, Furyl, Thiazolyl oder Thiadiazolyl, und $R_b$ und $R_c$ zusammen vorzugsweise O-substituiertes Hydroxyimino in der syn-Konfiguration bedeuten.

4. Verfahren nach Patentanspruch 3 zur Herstellung von Verbindungen der Formel I sowie Salzen von solchen Verbindungen mit salzbildenden Gruppen, dadurch gekennzeichnet, dass $R_1^a$ Wasserstoff oder eine Acylgruppe der Formel IA darstellt, worin (1) $R_a$ für Phenyl, 2- oder 3-Thienyl, 2- oder 3-Furyl, $R_b$ für Wasserstoff, freies oder, z.B. wie oben beschrieben, geschütztes Amino und $R_c$ für Wasserstoff stehen, oder worin (2) $R_a$ für Phenyloxy und $R_b$ und $R_c$ je für Wasserstoff stehen oder worin (3) $R_a$ Phenyl, 2-Amino-4-thiazolyl, 2-Niederalkylamino-, z.B. 2-Methylamino-4-thiazolyl, 5-Amino-1,2,4-thiadiazolyl oder 5-Niederalkylamino, z.B. 5-Methylamino-1,2,4-thiadiazolyl, worin Amino z.B. durch Acyl, wie gegebenenfalls halogeniertes Niederalkoxycarbonyl, z.B. tert.-Butyloxycarbonyl oder 2,2,2-Trichloräthoxycarbonyl, geschützt sein kann, darstellt, und $R_b$ und $R_c$ zusammen syn-Niederalkoxyimino, z.B. syn-Methoxyimino, bedeuten, und $R_2$ Hydroxy, eine veresterte Hydroxygruppe, beispielsweise Niederalkoxy, z.B. Methoxy oder tert.-Butoxy, 2-Halogenniederalkoxy, z.B. 2,2,2-Trichloräthoxy, Nitrobenzyloxy, z.B. 4-Nitrobenzyloxy, oder Diphenylmethoxy bedeutet.

5. Verfahren nach einem der Patentansprüche 1-3, dadurch gekennzeichnet, dass man 7β-Phenyloxyacetylamino-3-cephem-4-carbonsäure herstellt.

6. Verfahren nach einem der Patentansprüche 1-3, dadurch gekennzeichnet, dass man 7β-Phenylacetylamino-3-cephem-4-carbonsäure herstellt.

7. Verfahren nach einem der Patentansprüche 1-3, dadurch gekennzeichnet, dass man 7β-Amino-3-cephem-4-carbonsäure herstellt.

8 . Verfahren nach einem der Patentansprüche 1-3, dadurch gekennzeichnet, dass man 7β-Amino-3-cephem-4-carbonsäure-t.butylester herstellt.

9. Verfahren nach einem der Patentansprüche 1-3, dadurch gekennzeichnet, dass man 7β-Amino-3-cephem-4-carbonsäurediphenylmethylester herstellt.

10. Verfahren nach einem der Patentansprüche 1-3, dadurch gekennzeichnet, dass man 7β-Amino-3-cephem-p-nitrobenzylester herstellt.

11. Verfahren nach einem der Patentansprüche 1-11, dadurch gekennzeichnet, dass man komplexe Borhydride verwendet.

12. Verfahren nach Patentanspruch 11, dadurch gekennzeichnet, dass man Alkalimetallborhydride verwendet.

13. Verfahren nach Patentanspruch 12, dadurch gekennzeichnet, dass man Natriumborhydrid verwendet.

14. Verfahren nach Patentanspruch 12, dadurch gekennzeichnet, dass man Kaliumborhydrid verwendet.

15. Verfahren nach Patentanspruch 12, dadurch gekennzeichnet, dass man Natriumcyanborhydrid verwendet.

16. Verfahren nach einem der Patentansprüche 1-15, dadurch gekennzeichnet, dass man als saures Reagenz eine Carbonsäure, Dicarbonsäure oder Sulfonsäure verwendet.

17. Verfahren nach Patentanspruch 16, dadurch gekennzeichnet, dass man als Carbonsäure Eisessig verwendet.

18. Verfahren nach einem der Patentansprüche 11-17, dadurch gekennzeichnet, dass man komplexe Borhydride in Gegenwart von Eisessig verwendet.

19. Verfahren nach einem der Patentansprüche 1-15, dadurch gekennzeichnet, dass man als saures Reagenz nicht oxydierend oder reduzierend wirkende Lewis-Säuren verwendet.

20. Verfahren nach Anspruch 19, dadurch gekennzeichnet, dass man als Lewis-Säure Titan-(IV)-chlorid verwendet.

21. Verfahren nach Anspruch 19, dadurch gekennzeichnet, dass man als Lewis-Säure Kobalt(II)-chlorid verwendet.

22. Verbindungen der Formel

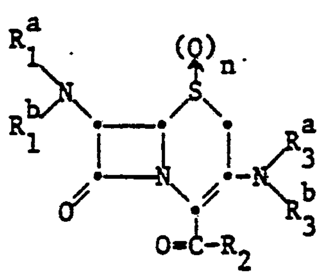

(II),

worin der Index n den Wert 1 hat, $R_1^a$ Wasserstoff oder eine Aminoschutzgruppe $R_1^A$, $R_1^b$ Wasserstoff oder eine Acylgruppe Ac oder $R_1^a$ und $R_1^b$ zusammen eine bivalente Aminoschutzgruppe, $R_2$ Hydroxy oder einen zusammen mit der Carbonylgruppierung -C(=O)- eine geschützte Carboxylgruppe bildenden Rest, und die Gruppe der Formel $-N(R_3^a)(R_3^b)$ eine sekundäre oder tertiäre Aminogruppe darstellt, sowie Salze von Verbindungen der Formel I mit salzbildenden Gruppen.

23. Verfahren zur Herstellung von Verbindungen der Formel

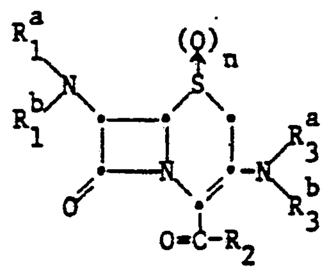

(II),

worin der Index n den Wert 1 hat, $R_1^a$ Wasserstoff oder eine Aminoschutzgruppe $R_1^A$, $R_1^b$ Wasserstoff oder eine Acylgruppe Ac oder $R_1^a$ und $R_1^b$ zusammen eine bivalente Aminoschutzgruppe, $R_2$ Hydroxy oder einen zusammen mit der Carbonylgruppierung -C(=O)- eine geschützte Carboxylgruppe bildenden Rest und die Gruppe der Formel $-N(R_3^a)(R_3^b)$ eine sekundäre oder tertiäre Aminogruppe darstellt, sowie Salzen von Verbindungen der Formel I mit salzbildenden Gruppen, dadurch gekennzeichnet, dass man eine Verbindung der Formel

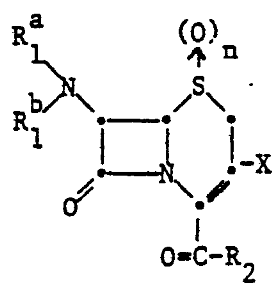

(III),

worin der Index n den Wert 1 hat und X Hydroxy, Chlor, Brom oder eine Sulfonyloxygruppe der Formel $-O-SO_2-R_4$ bedeutet und $R_1^a$, $R_1^b$ und $R_2$ die unter Formel I genannten Bedeutungen haben und worin, falls X Hydroxy bedeutet, $R_2$ nicht Hydroxy sein kann, mit einem primären oder sekundären Amin der Formel $H-N(R_3^a)(R_3^b)$, worin $R_3^a$ und $R_3^b$ die unter Formel II genannten Bedeutungen haben, umsetzt und, wenn erwünscht, ein erhältliches Salz in die freie Verbindung oder in ein anderes Salz und/oder eine erhältliche freie Verbindung mit einer salzbildenden Gruppe in ein Salz überführt.